(19) Europäisches Patentamt
European Patent Office
Office européen des brevets

(11) EP 2 124 872 B1

(12) EUROPEAN PATENT SPECIFICATION

(45) Date of publication and mention of the grant of the patent:
**06.06.2018 Bulletin 2018/23**

(21) Application number: **08723179.1**

(22) Date of filing: **27.02.2008**

(51) Int Cl.:
***A61K 8/34*** (2006.01) ***A61Q 19/02*** (2006.01)
***A61Q 19/08*** (2006.01)

(86) International application number:
**PCT/KR2008/001141**

(87) International publication number:
**WO 2008/105632 (04.09.2008 Gazette 2008/36)**

# (54) IMPROVING SKIN CONDITIONS WITH ALPHA-BISABOLOL AS ACTIVE PRINCIPLE

VERBESSERUNG DES HAUTZUSTANDES MIT ALPHA-BISABOLOL ALS WIRKSTOFF

AMÉLIORATION DES CONDITIONS CUTANÉES AVEC L'ALPHA-BISABOLOL EN TANT QUE PRINCIPE ACTIF

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.02.2007 KR 20070019899**

(43) Date of publication of application:
**02.12.2009 Bulletin 2009/49**

(73) Proprietor: **Biospectrum, Inc.**
**Gunpo-city, Gyeonggi-do 435-776 (KR)**

(72) Inventors:
- **PARK, Deok-Hoon**
  **Yongin-si**
  **Gyeonggi-do 448-171 (KR)**
- **KIM, Sae-Bom**
  **Seongnam-si**
  **Gyeonggi-do 463-922 (KR)**
- **LEE, Jong-Sung**
  **Anyang-si**
  **Gyeonggi-do 430-710 (KR)**

(74) Representative: **Scholz, Volker et al**
**Boehmert & Boehmert**
**Anwaltspartnerschaft mbB**
**Pettenkoferstrasse 22**
**80336 München (DE)**

(56) References cited:
**EP-A1- 1 582 202** **WO-A1-01/47481**
**WO-A1-98/34591** **WO-A2-00/62741**
**WO-A2-00/62744** **WO-A2-02/36090**
**WO-A2-02/051360** **WO-A2-02/087518**
**DE-A1-102005 010 142** **GB-A- 2 207 051**

- **BASF: "Bisabolol", Technical Information, May 2005 (2005-05), XP002662972, MEMC 050401e-00/Pages 1-8 Retrieved from the Internet: URL:http://www.basf.cl/carechemicals/cosmetica/fichastecnicas/ingredientesactivos/bisabolol.pdf [retrieved on 2011-11-07]**
- **RUBERTO G ET AL: "ANTIOXIDANT ACTIVITY OF SELECTED ESSENTIAL OIL COMPONENTS IN TWO LIPID MODEL SYSTEMS", FOOD CHEMISTRY, ELSEVIER LTD, NL, vol. 69, no. 2, 1 January 2000 (2000-01-01), pages 167-174, XP001029732, ISSN: 0308-8146, DOI: 10.1016/S0308-8146(99)00247-2**
- **MCKAY D.L. ET AL.: 'A Review of the Bioactivity and Potential Health Benefits of Chamomile Tea (Matricaria recutita L.)' PHYTOTHERAPY RESEARCH vol. 20, 20 April 2006, pages 519 - 530, XP009122297**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 124 872 B1

## Description

### FIELD OF THE INVENTION

The present invention relates to a composition comprising alpha-bisabolol as active ingredients, exhibiting an excellent improvement effect on skin conditions, immune regulatory effect and anti-obesity effect with stability and safety.

### DESCRIPTION OF THE RELATED ART

The color of human skin is ascribed mainly to the concentration and distribution of melanin. Melanin is one of phenol-based high molecular weighted biosubstances and plays an important role in preventing skin damage elicited by UV. The tyrosinase activity present in melanocyte has reported as a pivotal factor for melanin biosynthesis, tyrosinase plays pivotal role in skin darkening process by converting tyrosine into DOPA and DOPA-quinone, which are intermediate product of melanin polymer generation.

Generally, among several intricate causes such as moisture content of skin, collagen content and immune responsiveness to external environments, the main factor of wrinkle formation involves the expression and activity of collagenase, a collagen-degrading enzyme to reduce synthesis and content of collagen.

Hormone imbalance has become increasingly worse due to environmental pollution and auto exhausts. Because of this, the incidence rate of hair loss has become higher, the incidence age is lowered and a variety of skin diseases such as atopy and psoriasis have occurred due to inducing disharmony of skin immune system. In addition, the number of young obesity patients has rapidly went on increasing because of the change of dietary life such as meat and instant food centered diets.

WO 00/62741 discloses skin care compositions containing a combination of skin care actives.

WO 00/62744 discloses skin care compositions containing combination of skin care actives.

WO 02/36090 A2 discloses cosmetic plaster for bleaching the skin.

WO 98/34591 discloses skin lightening compositions.

WO 01/47481 A1 discloses a composition for the treatment of dandruff.

GB 2207051 A discloses a composition based on hydroxypyridone derivatives for reducing hair loss.

DE 10 2005 010 142 A1 dislcoses agents for activating hair growth.

Roberto et al., Food Chemistry, 2000, 69, 167, discloses antioxidant activity of selected essential oil components in two lipid model systems.

McKay et al., Phytotherapy Research, 2006, 20, 519, discloses a review of the bioactivity and potential health benefits of camomile tea.

Accordingly, studies have been intensively made to develop substances capable of effectively resolving several phenomena (*i.e.*, melasma or freckles, incidence of wrinkles by UV, obesity, immune imbalance or hair loss) which have became serious social problems.

Throughout this application, several patents and publications are referenced and citations are provided in parentheses. The disclosure of these patents and publications is incorporated into this application in order to more fully describe this invention and the state of the art to which this invention pertains.

### DETAILED DESCRIPTION OF THIS INVENTION

The present inventors have made intensive researches to develop active substances having activities of improvement in skin whitening, wrinkle, UV-induced skin damage, and prevention in hair loss, oxidation and obesity with high stability and safety without side effects on skin. As a result, the present inventors have found that compositions comprising $\alpha$-bisabolol as an active ingredient allowed to provide compositions for improving skin conditions, anti-oxidation and anti-obesity, having excellent effects and safety.

Accordingly, it is an object of this invention to provide a composition for improving skin condition, wherein the skin condition is whitening, wrinkles, UV-induced skin damage or hair growth.

It is another object of this invention to provide a composition for anti-oxidation.

It is still another object of this invention to provide a composition for anti-obesity.

It is another object of this invention to provide a method for improving skin conditions.

It is still another object of this invention to provide a method for preventing oxidation.

It is another object of this invention to provide a method for suppressing obesity.

Other objects and advantages of the present invention will become apparent from the detailed description to follow taken in conjugation with the appended claims.

The above objects are achieved in accordance with the subject-matter of the independent claims. Preferred embodiments result from the sub-claims.

Described herein is a composition for improving skin condition comprising α-bisabolol as an active ingredient, wherein the skin condition is wrinkles, whitening, UV-induced skin damage or hair growth.

Further described herein is a method for improving skin condition, which comprises administering to a subject a composition comprising α-bisabolol as an active ingredient.

In one aspect of the present invention, there is provided a of a cosmetic composition for skin whitening or treating wrinkles, said composition comprising α-bisabolol as the only active ingredient for skin whitening or treating wrinkles.

Further described herein is a composition for anti-oxidation comprising α-bisabolol as an active ingredient.

Further described herein is a method for preventing oxidation, which comprises administering to a subject a composition comprising α-bisabolol as an active ingredient.

Further described herein is a use of α-bisabolol for manufacturing a composition for anti-oxidation.

Further described herein is a composition for anti-obesity comprising α-bisabolol as an active ingredient.

Further described herein is a method for suppressing obesity, which comprises administering to a subject a composition comprising α-bisabolol as an active ingredient.

Further described herein is a use of α-bisabolol for manufacturing a composition for anti-obesity.

The present inventors have made intensive researches to develop active substances having activities of improvement in skin whitening, wrinkle, UV-induced skin damage, and prevention in hair loss, oxidation and obesity with high stability and safety without side effects on skin. As a result, the present inventors have found that compositions comprising α-bisabolol as an active ingredient allowed to provide compositions for improving skin conditions, anti-oxidation and anti-obesity, having excellent effects and safety.

α-Bisabolol used as an active ingredient in the present invention is a main active ingredient of chamomile essential oil and monocyclic sesquiterpene alcohol. IUPAC name of α-bisabolol is (2S)-6-methyl-2-[(1R)-4-methyl-1-cyclohex-3-enyl]hept-5-en-2ol, and a compound derived from plants, represented by the following formula I. Chamomile has been generally known to be anti-inflammatory effect, sedative activity and alleviation effect on pain. In addition, bisabolol as its main component is colorless and transparent liquid having anti-inflammatory effect, wound healing and antimicrobial effect in a cosmetic formulation and has been used to various skin care products.

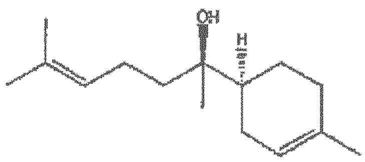
(I)

α-Bisabolol used as active ingredients in compositions of this invention may be extracted from natural source, chamomile such as Satureja punctata L or synthesized chemically. In detail, the α-bisabolol may be obtained using conventional various extraction methods. Preferably, the α-bisabolol may be obtained using various extraction solvents, *e.g.,* in 0°C to 50°C of extraction temperature, (a) water, (b) absolute or hydrous lower alcohol containing 1-4 carbon atoms (methanol, ethanol, propanol, butanol, etc.), (c) mixture of lower alcohol and water, (d) acetone, (e) ethyl acetate, (f) chloroform or (g) 1,3-butyleneglycol.

If necessary, α-bisabolol used in the present invention may be subjected to additional purification by the well-known methods in the art as well as those obtained by extraction. For instance, it could be appreciated that α-bisabolol obtained using a variety of additional purification methods such as ultrafiltration with defined molecular weight cut-off value and various chromatography (designed for purification dependent upon size, charge, hydrophobicity and affinity) may be used in the present invention.

The compositions of the present invention have novel use of skin whitening. The term "whitening" used herein, refers to improvement of skin trouble preventing skin melanism induced by melanin pigmentation.

In the composition for skin whitening of this invention, α-bisabolol exhibits the inhibition effect on melanin production by inhibiting intracellular tyrosinase activity compared to arbutin used as an existing skin whitening agent, and as a result, excellent efficacy in skin whitening. α-Bisabolol shows a high stability and no or a little side effects such as skin irritability induction. Furthermore, the content of α-bisabolol is constantly maintained in the composition. Such effects and efficacies are demonstrated in Examples described hereunder.

In addition, the compositions for improving skin condition of the present invention have novel use to improve skin wrinkles. The compositions of the present invention exhibit the inhibition effect on collagenase activity and promotion effect on collagen biosynthesis at a molecular level, and as a result, excellent efficacy in improvement of skin wrinkles. Such effects and efficacies are demonstrated in Examples described hereunder. It would be appreciated that the improvement of skin wrinkles covers general uses of skin protection (*e.g.*, prevention of skin wrinkles, removal of skin wrinkles and prevention of skin aging).

Moreover, the compositions for improving skin condition of this invention have alleviating, improving, preventing or treating effects on UV-induced skin damage.

The compositions for improving skin conditions of this invention contribute very effectively to the prevention of hair loss or the promotion of hairs growth. The terms "hair loss prevention" and "hairs growth promotion" used herein have the same meaning.

α-Bisabolol as an active ingredient of the present compositions exhibit an anti-oxidation effect by eliminating free radicals effectively.

The composition for anti-oxidation of the present invention may be applied to a variety of diseases, disorders or abnormal conditions capable of preventing or treating by inhibiting or eliminating oxidation conditions. The diseases associated with anti-oxidation to be treated by the present composition include neurodegenerative disorders such as atherosclerosis, coronary heart disease, restenosis, reperfusion injury, parkinson's disease or alzheimer's disease, stroke, cancer, aging, cardiovascular disorder, osteoporosis, disorder of central nervous system, peripheral vascular disease and dyspnea, but not limited to. Most preferably, the composition for anti-oxidation of this invention is used to prevention or remedy of aging. The correlation between various disorders and free radical damage has been reported generally and the component of various cells comprising enzyme, ion channel, structural protein and membrane lipid is a potential target against reactive free radical species (Rice-Evans C, Mol Aspects of Med 13(1):1-111(1992)). The reaction of the potential target and free radicals impairs cell function of specific ranges, induces pathological changes and allows for cell death ultimately. A variety of diseases caused by physiological oxidation are disclosed in U.S. Pat. No 5750351; 5773209; 5773231 and 5846959.

The composition for anti-oxidation of this invention protects DNA, protein (including lipoprotein) and membrane lipid from oxidative damage by excellent effects on elimination of free radicals.

Moreover, α-bisabolol as an active ingredient of this invention has an excellent anti-obesity effect.

It is obvious to one skilled in the art that α-bisabolol represented by Formula I includes derivatives obtained by chemical processes with substituents performed conventionally in one skilled in the art. The derivatives show the effects of skin whitening by inhibition of melanin synthesis and/or tyrosinase activity, or improvement in wrinkles and UV-induced skin damage, hair growth promotion or hair loss prevention, anti-oxidation and anti-obesity effects. More particularly, α-bisabolol used as an active ingredient of this invention includes derivatives of Formula I as well as compounds of Formula I. The derivatives with structure of Formula I as nucleus may be obtained by chemical processes using various substituents well-known in the art. For example, it would be suggested that derivatives formed by linking hydroxyl, halo, nitro or $C_{1-3}$ alkyl group to Formula I are likely to exert effects identical or similar to α-bisabolol. These substituted derivatives may be also fall into the scope of this invention.

According to a preferred embodiment, the active ingredient of α-bisabolol in the composition is present in the amount of 0.00001-15.0 wt%, more preferably, 0.0001-10 wt%, most preferably, 0.0001-5 wt% based on the total weight of the composition. If the amount of the active ingredient of α-bisabolol is lower than 0.00001 wt%, the effect of the composition may be negligible; in the case of exceeding 15.0 wt%, some adverse effects such as skin irritation and instability in formulation are very likely to occur.

The composition of the present invention is a cosmetic composition.

The cosmetic compositions of the present invention may contain auxiliaries as well as carrier in addition to the α-bisabolol as an active ingredient. The non-limiting examples of auxiliaries include antioxidants, stabilizers, solubilizers, vitamins, colorants, odor improvers or mixtures of these ingredients. In addition, the cosmetic compositions may additionally comprise promoting materials of skin absorption to enhance the effects.

The cosmetic compositions of this invention may be formulated in a wide variety of form, for non-limited example, including a solution, a suspension, an emulsion, a paste, a gel, a cream, a lotion, a powder, a soap, a surfactant-containing cleanser, an oil, a powder foundation, an emulsion foundation, a wax foundation and a spray. In detail, the cosmetic composition of the present invention can be provided in a form of skin softener (skin lotion), nutrient emulsion (milk lotion), nutrient cream, message cream, essence, eye cream, cleansing cream, cleansing foam, cleansing water, facial pack, spray or powder.

The cosmetically acceptable carrier contained in the present cosmetic composition, may be varied depending on the type of the formulation. For example, the formulation of pastes, creams or gels may comprise animal and vegetable fats, waxes, paraffins, starch, tragacanth, cellulose derivatives, polyethylene glycols, silicones, bentonites, silica, talc, zinc oxide or mixtures of these ingredients.

In the formulation of powder or spray, it may comprise lactose, talc, silica, aluminum hydroxide, calcium silicate, polyamide powder or mixtures of these ingredients. Spray may additionally comprise the customary propellants, for example, chlorofluorohydrocarbons, propane/butane or dimethyl ether.

The formulation of solution and emulsion may comprise solvent, solubilizer or emulsifier, for example water, ethanol, isopropanol, ethyl carbonate, ethyl acetate, benzyl alcohol, benzyl benzoate, propylene glycol, 1,3-butyleneglycol oils, glycerol fatty esters, polyethylene glycol, fatty acid esters of sorbitan or mixtures of these ingredients.

The formulation of suspension may comprise liquid diluents, for example water, ethanol or propylene glycol,

suspending agents, for example ethoxylated isosteary alcohols, polyoxyethylene sorbitol esters and poly oxyethylene sorbitan esters, micocrystalline cellulose, aluminum metahydroxide, bentonite, agar and tragacanth or mixtures of these ingredients.

The formulation of cleansing compositions with surfactant may comprise aliphatic alcohol sulfate, aliphatic alcohol ether sulfate, sulfosucinnate monoester, isothinate, imidazolium derivatives, methyltaurate, sarcocinate, fatty acid amide ether sulfate, alkyl amido betain, aliphatic alcohol, fatty acid glyceride, fatty acid diethanolamide, vegetable oil, lanoline derivatives, ethoxylated glycerol fatty acid ester or mixtures of these ingredients.

The composition of this invention may be prepared as a pharmaceutical composition, and the pharmaceutically acceptable carrier as well as the active ingredient contained in the pharmaceutical composition. The pharmaceutically acceptable carrier, which is commonly used in pharmaceutical formulations, but is not limited to, includes lactose, dextrose, sucrose, sorbitol, mannitol, starch, rubber arable, potassium phosphate, arginate, gelatin, potassium silicate, microcrystalline cellulose, polyvinylpyrrolidone, cellulose, water, syrups, methyl cellulose, methylhydroxy benzoate, propylhydroxy benzoate, talc, magnesium stearate, and mineral oils. The pharmaceutical composition according to the present invention may further include a lubricant, a humectant, a sweetener, a flavoring agent, an emulsifier, a suspending agent, and a preservative. Details of suitable pharmaceutical acceptable carriers and formulations can be found in Remington's Pharmaceutical Sciences (19th ed., 1995*).*

The pharmaceutical composition of this invention may be administered to mammals such as rat, mouse, domestic animals and human *via* various routes, for example oral administration, rectal administration or intravenous injection, intramuscular injection, subcutaneous injection, intrauterine injection or intracerebroventricular injection, preferably subcutaneous injection, more preferably topical application.

A suitable dosage amount of the pharmaceutical composition of the present invention may vary depending on pharmaceutical formulation methods, administration methods, the patient's age, body weight, sex, pathogenic state, diet, administration time, administration route, an excretion rate and sensitivity for a used pharmaceutical composition, and physicians of ordinary skill in the art can determine an effective amount of the pharmaceutical composition for desired treatment. In case of oral formulation, a suitable dosage unit may be administered once to several times a day with 0.001-100 mg/kg on the basis of adult. In case of preparation for external use, a suitable dosage unit may be administered by applying once to five times a day in amounts of 1.0 to 3.0 ml on the basis of adult and it has better use for more than 1 month. However, the dosage unit does not limit the scope of this invention.

According to the conventional techniques known to those skilled in the art, the pharmaceutical composition of the present invention may be formulated with pharmaceutically acceptable carrier and/or vehicle as described above, finally providing several forms a unit dose form and a multi-dose form. Non-limiting examples of the formulations include, but not limited to, oral formulation such as a powder, a granule, a tablet, a capsule, a suspension, an emulsion, a syrup and an aerosol, preparation for external use such as an ointment and a cream, a suppository and sterile injection solution, and may further comprise a dispersion agent or a stabilizer.

The composition of this invention may be prepared as a food composition. The food composition of this invention may comprise conventional additives for preparing food compositions, e.g., protein, carbohydrates, lipids, nutritive substances and flavors.

Non-limiting examples of carbohydrates described above include, but not limited to, monosaccharide (*e.g.*, glucose and fructose); disaccharide (e.g., maltose, sucrose and oligosaccharide); and polysaccharide (*e.g.*, dextrin and cyclodextrin); and sugar alcohol (e.g., xylitol, sorbitol and erithritol). Non-limiting examples of Flavors include, but not limited to, natural flavors [thaumatin and extract of *stevia* (*e.g.*, rebaudioside A and glycyrrhizin)] and synthetic flavors (*e.g.*, saccharin and aspartame).

For example, where the food composition of this invention is provided as a drink, it may further comprise citric acid, liquid fructose, sugar, glucose, acetic acid, malic acid, fruit juice, extract of eucommia ulmoides oliv, jujube extract or extract of glycyrrhiza uralensis.

Meanwhile, experiment results of a cumulative skin irritation elucidated that α-bisabolol as a natural substance was harmless to human body in specific example of the present invention. Therefore, α-bisabolol of the present invention may be used with confidence for long period due to not or a little having toxicities and side effects, particularly may be applied to cosmetic, pharmaceutical and food composition with safety as described above.

The features and advantages of this invention is summarized as follows:

(i) The composition of the present invention comprises α-bisabolol as an active ingredient.

(ii) α-Bisabolol has greater skin whitening effect inhibiting melanin synthesis by inhibiting intracellular tyrosinase activities compared to arbutin used as an existing skin whitening agent. In addition, α-bisabolol exhibits the inhibition effect on collagenase activity and promotion effect on collagen biosynthesis at a molecular level, contributing to excellent efficacy in improvement of skin wrinkles. Meanwhile, α-bisabolol indicates the improvement effects on UV-induced skin damage and the skin growth promotion or hair loss prevention. Therefore, α-bisabolol has the excellent improvement effects on skin condition.

(iii) Furthermore, α-bisabolol has the excellent anti-obesity and anti-oxidation effects.
(iv) The composition of this invention can be applied to cosmetic, pharmaceutical and food composition having no cytotoxicities and side effects with safety.

The present invention will now be described in further detail by examples. It would be obvious to those skilled in the art that these examples are intended to be more concretely illustrative and the scope of the present invention as set forth in the appended claims is not limited to or by the examples.

## EXAMPLES

### Example 1: Measurement of effects of α-bisabolol on inhibition of melanin production

After measuring the inhibition of melanin production by α-bisabolol using B16 mouse melanoma cells (Korean Cell Line Bank), the measurement was compared with that for the inhibition of melanin production by arbutin, known as a melanin production inhibitor.

B16 mouse melanoma cells F10 (Korean Cell Line Bank) were seeded into each well of a 6-well plate (1 x $10^5$ cells/well) in DMEM (Dulbecco's modified Eagle's media) containing 10% FBS (fetal bovine serum) and the cells were cultured to about more than 80% adherence by incubating in a $CO_2$ incubator under conditions of 37°C and 5.0% $CO_2$. After cultivation, the medium was removed and samples were replaced in medium diluted at suitable concentration, followed by incubating for 3 days under conditions of 37°C and 5.0% $CO_2$. The concentration of α-bisabolol was determined with 1 ppm, 5 ppm and 10 ppm, which did not show cytotoxicity. The cells removing medium were washed with PBS (phosphate buffer saline) and treated with trypsin to collect cells. The number of the collected cells was calculated using hematocytometer (Tiefe Depth Profondeur 0.100 mm, Paul Marienfeld GmbH & Co. KG, Germany), centrifuged at 5,000 to 10,000 rpm for 10 min and the supernatant was eliminated, thereby obtaining pellets. This cell pellets were dried at 60°C, 100 μl of 1M NaOH containing 10% DMSO was added to thereto, and intracellular melanin was obtained in incubator at 60°C. Then, the cell solution was measured for absorbance at 490 nm using microplate reader (Bio-Tek ELx8081U, U.S) and the amount of melanin per cell constant number was estimated. The experiment results were summarized in Table 1.

**TABLE 1**

| Samples | Treatment concentration (ppm) | Inhibitory rate of melanin production (%) |
|---|---|---|
| Arbutin | 5 | 22 |
| α-Bisabolol | 1 | 24 |
| | 5 | 36 |
| | 10 | 49 |

As indicated in Table 1, even where the treatment concentration of α-bisabolol was lower than that of arbutin, α-bisabolol had higher inhibitory rate of melanin production. In addition, it was founded that the inhibitory rate of melanin production went on increasing as the treatment concentration of α-bisabolol increased.

### Example 2: Inhibitory effect on tyrosinase activity by α-bisabolol

After measuring the inhibition of melanin production by α-bisabolol using B16 mouse melanoma cells (Korean Cell Line Bank), the measurement was compared with that for the inhibition of intracellular tyrosinase activities by arbutin, known as a melanin production inhibitor.

Murine melanoma (B-16 F1) cells were seeded into each well of a 6-well plate (1 x $10^5$ cells/well) in DMEM containing 10% FBS (fetal bovine serum) and the cells were cultured to about more than 80% adherence by incubating in a $CO_2$ incubator under conditions of 37°C and 5.0% $CO_2$. After cultivation, the medium was removed and samples were replaced in medium diluted at suitable concentration, followed by incubating for 3 days under conditions of 37°C and 5.0% $CO_2$. The concentration of α-bisabolol was determined with 1 ppm, 5 ppm and 10 ppm, which did not show cytotoxicity. The cells removing medium were washed with PBS (phosphate buffer saline) and treated with trypsin to collect cells. The number of the collected cells was calculated using hematocytometer, centrifuged at 5,000 to 10,000 rpm for 10 min and the supernatant was eliminated, thereby obtaining pellets. This cell pellets were lysated using lysis buffer, centrifuged at 12,000 rpm for 10 min and the supernatant was collected. Then, the cell solution was measured for absorbance at 492 nm using microplate reader and the activity of tyrosinase per cell constant number was estimated.

The results were summarized in Table 2.

**TABLE 2**

| Sample | Treatment concentration (ppm) | Inhibitory rate of intracellular tyrosinase activity (%) |
|---|---|---|
| Arbutin | 5 | 45 |
| α-Bisabolol | 1 | 13 |
| | 5 | 38 |
| | 10 | 58 |

As shown in Table 2, the results demonstrate that α-bisabolol inhibited significantly greater effects on the inhibition of intracellular tyrosinase activities than arbutin. In addition, α-bisabolol exhibited the inhibition effects on tyrosinase activities in concentration-dependant manner.

**Example 3: Evaluation of skin whitening effect in animal**

A skin whitening effect of α-bisabolol was measured using brown guinea pigs (Charles River Laboratories, Inc.) pigmentation of which is known to increase upon exposure to ultraviolet light like human.

To induce pigmentation in the brown guinea pig by ultraviolet (UV), aluminum foil with square windows of 3 x 3 $cm^2$ was adhered to hair-removed abdominal skin of brown guinea pig, and then UV light was irradiated thereon with a SE lamp (wavelength 290-320 nm, Toshiba) (total irradiation energy = 1350 $mJ/cm^2$). After UV irradiation, the aluminum foil was removed and samples (α-bisabolol or arbutin) were applied as the following method. Increased pigmentation was observed on 2-3 days after UV irradiation and reached a maximum after about 2 weeks. Following the period of time showing the maximum pigmentation, samples were applied.

The application of samples was performed once or twice a day for 50 days. The samples were dissolved or diluted in a solvent (Propylene glycol : ethanol : water = 5 : 3 : 2) and applied by a swab. The control with only the solvent was applied to another site. The occurrence of cumulative irritation was also examined.

The degree of pigmentation of skin was determined using a chromameter (CR2002, MINOLTA, JP) to estimate the effects of applied samples. The results are shown in Table 3 below. L*a*b* colorimetric system was used to classify color and L* value was used as standard in Example. The L* value was corrected using white board standard and was measured more than five times at one site. Pigmentation was evenly distributed. Skin color differences (ΔL*) between application starting point and application end point were obtained and used to evaluate effects of samples. The results were summarized in Table 3.

**Equation 1**

$$\Delta L^* = L^* \text{ value on 00 days after application} - L^* \text{ value on application starting day}$$

ΔL* values were obtained for control and sample application to evaluate skin whitening effects.

**TABLE 3**

| Samples | Treatment concentration (%) | Whitening effects (ΔL) |
|---|---|---|
| α-Bisabolol | 0.2 | 0.42 |
| | 1.0 | 0.67 |
| Arbutin | 1.0 | 0.54 |
| Control | - | 0.35 |

As described in Table 3, α-bisabolol exhibited the whitening effects in concentration-dependant manner. Furthermore, it was revealed that α-bisabolol had more excellent whitening effects than arbutin as well as considerable safety that was verified by no observation of cumulative irritation.

**Example 4: Safety test of α-bisabolol on human skin**

**Example 4-1: Preparation of a skin external composition containing α-bisabolol**

A skin external composition containing α-bisabolol and arbutin was prepared and its safety test on human skin was performed to determine whether α-bisabolol is safe on skin.

The skin external composition containing α-bisabolol and arbutin was prepared as indicated in Table 4. The control group in Table 4 is a skin external composition not containing tyrosinase inhibitor, the test groups 1 and 2 are a skin external composition containing α-bisabolol and arbutin, respectively.

To prepare a skin external composition, distilled water, serine and butylene glycol were mixed and dissolved at 70°C (water phase) and the remaining components were dissolved at 70°C (oil phase). The oil phase was added to the water phase and agitated using a homomixer (Tokushu Kika, Japan) for first emulsification, followed by addition of trimethanolamine. The air bubble formed in the mixed solution was eliminated and cooled to room temperature to prepare the skin external composition.

**TABLE 4**

| Components | Contents (wt%) | | |
|---|---|---|---|
| | Control | Test 1 | Test 2 |
| Purified water | 72 | 72 | 72 |
| Glycerin | 8.0 | 8.0 | 8.0 |
| Butyleneglycol | 4.0 | 4.0 | 4.0 |
| Alpha-bisabolol | 0 | 1.0 | 0 |
| Arbutin | 0 | 0 | 1.0 |
| Caprylic capric triglyceride | 8.0 | 8.0 | 8.0 |
| Squalene | 5.0 | 5.0 | 5.0 |
| Cetearyl glucoside | 1.5 | 1.5 | 1.5 |
| Sorbitan stearate | 0.4 | 0.4 | 0.4 |
| Cetearyl alcohol | 1.0 | 1.0 | 1.0 |
| Trimethanolamine | 0.1 | 0.1 | 0.1 |
| Total | 100 | 100 | 100 |

**Example 4-2: Cumulative skin irritation test**

Each skin external composition prepared in Example 4-1 was applied in patches 9 times to the upper arms of 30 healthy adults once every other day for a total time period of 24 hours. This 24-hour cumulative patch test was conducted to determine whether α-bisabolol irritates the skin or not.

The Finn chamber (Epitest Ltd, Finland) was chosen for the patching method. The above preparation for external use on the skin was dropped into each chamber in an amount of 15 μl, and a patch was applied. The response level on the skin for each test was scored using the Equation 2, and the result was shown in Table 5.

**Equation 2**

Average response level = [{(response index x response level)/No. of test subjects x maximum points (4 points)} x 100] ÷ No. of test (9 times)

Points were marked in accordance with response level, for example, ± for 1 point, + for 2 points, and ++ for four points. The composition can be considered safe if the average response level is less than 3.

**TABLE 5**

| Test materials | No. of testee with response | | | | | | Average response level | | | |
|---|---|---|---|---|---|---|---|---|---|---|
| | Week 1 | | Week 2 | | Week 3 | | | | | |
| | 1st | 2nd | 3rd | 4th | 5th | 6th | 7th | 8th | 9th | |
| | ± + + + | ± + + + | ± + + + | ± + + + | ± + + + | ± + + + | ± + + + | ± + + + | ± + + + | |
| Control | 2 - - | - - - | - - - | - - - | - - - | - - - | - - - | - - - | - - - | 0.18 |
| Test group 1 | 3 - - | 1 - - | - - - | - - - | - - - | - - - | - - - | - - - | - - - | 0.37 |
| Test group 2 | 3 - - | 1 - - | - - - | - - - | - - - | - - - | - - - | - - - | - - - | 0.37 |
| No. of testee | 30 | 30 | 30 | 30 | 30 | 30 | 30 | 30 | 30 | - |

In Table 5, the number of testee is 3, zero, and 1, respectively, for ±, + and ++ in Test group 1 and 2, and no further responses were shown. The average response level was calculated to be not more than 3 (*i.e.*, 0.37) according to Equation 2, demonstrating that the compositions are safe.

As shown in Table 5, the composition comprising α-bisabolol (Test group 1) did not show any significant cumulative irritation as either control group or the arbutin-containing composition. Therefore, it could be appreciated that α-bisabolol is a safe substance for human skin.

**Example 5: Anti-oxidation effect**

Superoxide dismutase (SOD) has been known as an anti-oxidation-catalyzing enzyme which converts superoxide anion into $H_2O_2$ and $O_2$. This experiment evaluates anti-oxidation activity of samples by observing removal of superoxide anion generated by xanthine oxidase. The experiment was performed using the kit (SOD Assay Kit-WST) purchased from Dojindo (JP) in accordance with manufacture's protocol. The experiment results were summarized in Table 6.

**TABLE 6**

| Samples | Treatment concentration (ppm) | Removal rate of superoxide anion (%) |
|---|---|---|
| α-Bisabolol | 1 | 8 |
| | 5 | 18 |
| | 10 | 34 |

As shown in Table 6, α-bisabolol of the present invention exerted the anti-oxidation effects in concentration-dependant manner.

**Example 6: Evaluation of anti-inflammatory effects**

To determine whether α-bisabolol had anti-inflammatory effects, the experiment of COX (cyclooxygenase) inhibitory ability was carried out using human monocytic cell, THP-1 cell (Korean Cell Line Bank) according to conventional method. The COX activity was measured in accordance with Methods in Enzymology 43:9 (1994) published by F. J. Van de Ouderaaa and Muytenhek. THP-1 cell line was cultivated and aliquoted into 24-well plate. The incubation volume per well was adjusted to 500 μl, 2 μl of the sample compound, which dissolved in lipopolysaccahride (1 μg/ml, Sigma) and solvents described in the following Table respectively, was added and cultivated for 24-48 hr under the same condition. After 24-48 hr, calcium ionophore (Sigma) and [1-14C] arachidonic acid 1 μl (in EtOH, 0.1 μCi/ml, Sigma) were added to each well, and cultivated for 10 min under the same condition. Following the cultivation, citric acid was added to each well for adjusting to pH 3.5, shaked, each 500 μl of cultivation solution taken from the plate was aliquoted into micro centrifuge tube, 700 μl of ethylacetate was added to thereto, and the solution was shaking extracted for 10 min. 500 μl of ethylacetate layer was concentrated using speed vacuum dryer for 20 min, 20 μl of the residual was dissolved in ethylacetate, and the resultant was employed with authentic standard in TLC plate. The radioactive band was identified by authentic eicosanoid standards, the radioactivity of the identified band was measured using BAS 2000 bio-imaging analyzer (Fuji, JP) (Table 7).

**TABLE 7**

| Samples | COX activities (%) |
|---|---|
| LPS (1 $\mu$g/ml) | 100 |
| LPS(1 $\mu$g/ml)+ $\alpha$-bisabolol (1 ppm) | 95 |
| LPS(1 $\mu$g/ml)+ $\alpha$-bisabolol (5 ppm) | 75 |
| LPS(1 $\mu$g/ml)+ $\alpha$-bisabolol (10 ppm) | 67 |

As described in Table 7, the results address that $\alpha$-bisabolol effectively inhibits COX activities as treatment concentrations increased. From these results, it was founded that $\alpha$-bisabolol had the inflammatory inhibitory effects.

**Example 7: Evaluation of Immunosuppressive effects**

For examining whether the samples used in this example suppressed the immune response related to inflammatory, the experiment of interleukin-2 luciferase reporter activity was performed. Interleukin-2 promoter was reported to play an important role in the generation of cytokine related to inflammatory. The activity of interleukin-2 luciferase was measured using the following method: Human T lymphocytes cell line, 1 x $10^6$ of Jurkat cell (Korean Cell Line Bank) were aliquoted into each well of 6-wells, IL-2 luciferase reporter plasmid DNA (Stratagene) was transfected using superfect transfection reagent (In vitrogen). 24 hr after transfection, PHA (Phytohemaglutinin, Sigma) (100 ng/ml) was treated to activate Jurkat cell and each samples was treated with varying concentration. Following 24 hr, the cell was collected and the luciferase activity was measured using luminometer (Berthold Technologies GmbH&Co.KG, Germany). The results were summarized in Table 8.

**TABLE 8**

| Samples | IL-2 luciferase activities |
|---|---|
| PHA (100 ng/ml) | 100 |
| PHA(100 ng/ml)+ $\alpha$-bisabolol (1 ppm) | 92 |
| PHA(100 ng/ml)+ $\alpha$-bisabolol (5 ppm) | 72 |
| PHA(100 ng/ml)+ $\alpha$-bisabolol (10 ppm) | 58 |

As shown in Table 8, the results demonstrate that $\alpha$-bisabolol has the immunoregulatory activities by inhibiting the IL-2 expression as treatment concentrations increased.

**Example 8: Measurement of effect of $\alpha$-bisabolol on wrinkles improvement**

The wrinkle improvement effect can be generally evaluated by measuring collagen biosynthesis ability and collagenase degradation inhibitory ability and performing clinical test with human.

Human fibroblasts (commercially available from pacific) were seeded into a 6-well plate (2 x $10^5$ cells/well) and the well plate was incubated in a 5% $CO_2$ incubator for 24 hr at 37°C. After 24 hr, the medium in each well was removed and cells were incubated with various concentrations of samples for 24 hr. Afterwards, the cell medium was collected and was used as samples.

The potential of collagen synthesis was determined by measuring the amount of procollagen type I C-peptide (PICP) in cell medium using Procollagen Type I C-peptide EIA kit (MK101, Takara, Kyoto, Japan) in accordance with manufacturer's protocol.

For measuring the collagenase activity, antibodies against collagenase was used. The collagenase activity was measured using a Type 1 collagenase assay kit (Amersham Biosciences, RPN2629) and the absorbance values were obtain using an ELISA reader (Bio-Tek ELx808™ Series Ultra Microplate Reader, U.K). The measured average values were represented as mean $\pm$ standard deviation. A T-test with SPSS/PC+ was conducted to determine a statistical significance, and the result is shown in Table 9.

**TABLE 9**

| Test items | α-bisabolol (1 ppm) | α-bisabolol (5 ppm) | α-bisabolol (10 ppm) |
|---|---|---|---|
| Increase of collagen synthesis (%) | 23 | 43 | 76 |
| Inhibition of collagenase (%) | 12 | 28 | 43 |

As shown in Table 9, α-bisabolol enhanced collagen synthesis and inhibited collagenase activity in concentration-dependent manner. Therefore, these results demonstrate that α-bisabolol has a significant improvement effect on wrinkles.

**Example 9: Anti-obesity effect**

An obesity inhibition test was performed by the following method using well-known laboratory animals. The results are summarized in Table 10.

The process for determining obesity-inhibitory activity was as follows:

Crj : 7 week-old ICR male mice (obtained from Charles River Japan Ltd) weeks were preliminarily fed for 1 week, then classified into groups each having 7 animals and subjected to the test. The animals were fed under controlled conditions at a temperature of 23±1°C, and a humidity of 55±5% under illumination for 12 hours per day. They were fed with a feed Labo MR (manufactured by Nippon Nosan) and allowed to take water *ad libitum.* α-Bisabolol was present in the form of liposome (prepared with 5% lecithin) to the final concentration of either 0.1% or 1%. The concentration of each sample solution was adjusted so that 0.1 ml of the solution was given per 10 g body weight of mice. The doses employed were 1.5 g/kg and 1 g/kg. To a control group, 5% lecithin emulsion was administered. After fasting the mice, the sample was administered once by force on the next day. During the test period over 2 weeks, the body weight and general conditions were monitored.

**TABLE 10**

| Solvents | Days | Body weight (g) | | |
|---|---|---|---|---|
| | | Untreatment group | α-bisabolol (0.1%) | α-bisabolol (1%) |
| Liposome | 5 | 24 | 24 | 23 |
| | 10 | 28 | 24 | 24 |
| | 15 | 34 | 26 | 26 |

As shown in Table 10, the body weight gain was inhibited in α-bisabolol. The excellence of the effects was observed as administered concentration increased.

**Example 10: Effect of hair loss prevention and hairs growth promotion**

For measuring the effect of hair loss prevention and hairs growth promotion, the samples were prepared in the form of hydrogel base containing only viscosity-increasing agent and preservative, and test was performed. Each 3 cc of liquids for external use for promoting hairs growth prepared were applied to the hair loss sites of 10 baldness patients twice a day for 3 months. As a result, the liquids containing the samples showed an excellent effect such as the generation of the root of hair from 8 baldness patients. The experiment results were as follows. The control group used the moxidil commercially available from Hanmi pharmaceutical. Co. Ltd.

**TABLE 11**

| Untreatment group | Moxidil | α-bisabolol |
|---|---|---|
| - | ++ | ++ |

As indicated in Table 11, the results demonstrate that α-bisabolol of this invention exerted a similar hairs growth effect to commercially available moxidil as hairs growth formulations.

As described hereinabove, the present invention provides a composition for improving skin condition comprising α-bisabolol as an active ingredient. α-Bisabolol used as an active ingredient has greater skin whitening effect inhibiting melanin synthesis by inhibiting intracellular tyrosinase activities compared to arbutin used as an existing skin whitening

agent. In addition, $\alpha$-bisabolol exhibits the inhibition effect on collagenase activity and promotion effect on collagen biosynthesis at a molecular level, contributing to excellent efficacy in improvement of skin wrinkles. Meanwhile, $\alpha$-bisabolol indicates the improvement effects on UV-induced skin damage and the skin growth promotion or hair loss prevention. Therefore, $\alpha$-bisabolol has the excellent improvement effects on skin condition. Furthermore, $\alpha$-bisabolol has the excellent anti-obesity and anti-oxidation effects. The composition of this invention can be applied to cosmetic, pharmaceutical and food composition having no cytotoxicities and side effects with safety.

## Claims

1. Use of a cosmetic composition for skin whitening or treating wrinkles, said composition comprising $\alpha$-bisabolol as the only active ingredient for skin whitening or treating wrinkles.

2. Use of the cosmetic composition for skin whitening or treating wrinkles according to claim 1, wherein $\alpha$-bisabolol as the only active ingredient is present in the amount of 0.0001-10wt% based on the total weight of the cosmetic composition.

3. Use of the cosmetic composition for skin whitening or treating wrinkles according to claims 1 or 2, wherein the cosmetic composition is in the form of one selected from the group consisting of a solution, a suspension, an emulsion, a paste, a gel, a cream, a lotion, a powder, a soap, a surfactant-containing cleanser, an oil, a powder foundation, an emulsion foundation, a wax foundation and a spray.

4. Use of a food composition for skin whitening or treating wrinkles, said composition comprising $\alpha$-bisabolol as the only active ingredient for skin whitening or treating wrinkles.

## Patentansprüche

1. Verwendung einer kosmetischen Zusammensetzung zur Hautaufhellung oder zur Behandlung von Falten, besagte Zusammensetzung umfassend $\alpha$-Bisabolol als einzig aktiven Bestandteil zur Hautaufhellung oder zur Behandlung von Falten.

2. Verwendung der kosmetischen Zusammensetzung zur Hautaufhellung oder zur Behandlung von Falten nach Anspruch 1, wobei $\alpha$-Bisabolol als der einzig aktive Bestandteil in einer Menge von 0,0001-10 Gew.-%, bezogen auf das Gesamtgewicht der kosmetischen Zusammensetzung, enthalten ist.

3. Verwendung der kosmetischen Zusammensetzung zur Hautaufhellung oder zur Behandlung von Falten nach Anspruch 1 oder 2, wobei die kosmetische Zusammensetzung in einer Form vorliegt, ausgewählt aus der Gruppe, bestehend aus einer Lösung, einer Suspension, einer Emulsion, einer Paste, einem Gel, einer Creme, einer Lotion, einem Pulver, einer Seife, einem ein grenzflächenaktives Mittel enthaltenden Reiniger, einem Öl, einer Pulvergrundlage, einer Emulsionsgrundlage, einer Wachsgrundlage und einem Spray.

4. Verwendung einer Nahrungsmittelzusammensetzung zur Hautaufhellung oder zum Behandeln von Falten, besagte Zusammensetzung umfassend $\alpha$-Bisabolol als einzig aktiven Bestandteil zur Hautaufhellung oder zur Behandlung von Falten.

## Revendications

1. Utilisation d'une composition cosmétique pour blanchir la peau ou traiter les rides, ladite composition comprenant de l'$\alpha$-bisabolol en tant que le seul principe actif pour le blanchiment de la peau ou le traitement des rides.

2. Utilisation de la composition cosmétique pour blanchir la peau ou traiter les rides selon la revendication 1, dans laquelle l'$\alpha$-bisabolol en tant que le seul principe actif est présent en la quantité de 0,0001-10 % en poids sur la base du poids total de la composition cosmétique.

3. Utilisation de la composition cosmétique pour blanchir la peau ou traiter les rides selon les revendications 1 ou 2, où la composition cosmétique est sous forme d'un produit sélectionné parmi le groupe consistant en une solution,

une suspension, une émulsion, une pâte, un gel, une crème, une lotion, une poudre, un savon, un démaquillant contenant un tensioactif, une huile, un fond de teint en poudre, un fond de teint en émulsion, un fond de teint en cire et un atomiseur.

**4.** Utilisation d'une composition alimentaire pour blanchir la peau ou traiter les rides, ladite composition comprenant de l'$\alpha$-bisabolol en tant que le seul principe actif pour le blanchiment de la peau ou le traitement des rides.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0062741 A
- WO 0062744 A
- WO 0236090 A2
- WO 9834591 A
- WO 0147481 A1
- GB 2207051 A
- DE 102005010142 A1
- US 5750351 A
- US 5773209 A
- US 5773231 A
- US 5846959 A

### Non-patent literature cited in the description

- **ROBERTO et al.** *Food Chemistry,* 2000, vol. 69, 167
- **MCKAY et al.** *Phytotherapy Research,* 2006, vol. 20, 519
- **RICE-EVANS C.** *Mol Aspects of Med,* 1992, vol. 13 (1), 1-111
- Remington's Pharmaceutical Sciences. 1995
- Methods in Enzymology. F. J. Van de Ouderaaa and Muytenhek, 1994, vol. 43, 9